(19) Europäisches Patentamt
European Patent Office
Office européen des brevets

(11) EP 0 692 472 A1

(12) **DEMANDE DE BREVET EUROPEEN**

(43) Date de publication:
**17.01.1996 Bulletin 1996/03**

(51) Int Cl.[6]: **C07D 211/18**, A61K 31/445

(21) Numéro de dépôt: **95401658.0**

(22) Date de dépôt: **10.07.1995**

(84) Etats contractants désignés:
**AT BE CH DE DK ES FR GB GR IE IT LI LU NL PT SE**

(30) Priorité: **13.07.1994 FR 9408711**

(71) Demandeur: **SYNTHELABO**
**F-92350 Le Plessis Robinson (FR)**

(72) Inventeurs:
- **Thenot, Jean Paul**
**F-91190 Gif Sur Yvette (FR)**
- **Frost, Jonathan**
**F-91320 Wissous (FR)**
- **Lardenois, Patrick**
**F-92340 Bourg la Reine (FR)**
- **Renones, Maria Carmen**
**F-95880 Enghien Les Bains (FR)**
- **Wick, Alexander**
**F-78860 Saint Nom la Breteche (FR)**

(74) Mandataire: **Ludwig, Jacques et al**
**F-92352 Le Plessis Robinson Cédex (FR)**

(54) **Esters de alpha-(4-chlorophenyl)-4- (4-fluorophenyl)-methyl piperidine-1-ethanol, leur préparation et leur application en therapeutique**

(57) Composés de formule générale (I)

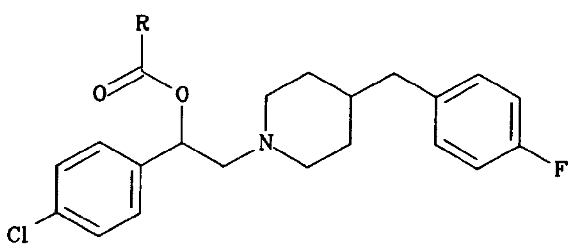

(I)

dans laquelle R représente un groupe ($C_1$-$C_{19}$)alkyle linéaire ou éventuellement ramifié et/ou contenant éventuellement une liaison éthylénique, un groupe ($C_3$-$C_6$)cycloalkyle, un groupe ($C_3$-$C_6$)cycloalkylméthyle, un groupe phényle éventuellement substitué par un atome d'halogène, ou un groupe phénylméthyle éventuellement substitué par un atome d'halogène.

Application en thérapeutique.

EP 0 692 472 A1

## Description

La présente invention a pour objet des esters de α-(4-chlorophényl)-4-[(4-fluorophényl)méthyl]pipéridine-1-éthanol, leur préparation et leur application en thérapeutique.

Les composés de l'invention répondent à la formule générale (I)

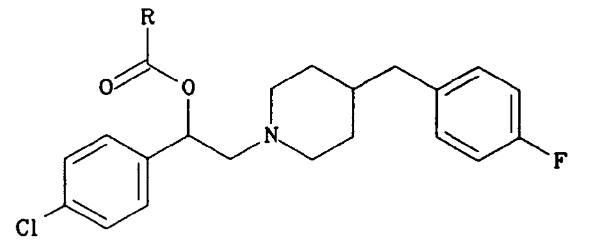

(I)

dans laquelle R représente un groupe $(C_1-C_{19})$alkyle linéaire ou éventuellement ramifié et/ou contenant éventuellement une liaison éthylénique, un groupe $(C_3-C_6)$cycloalkyle, un groupe $(C_3-C_6)$cycloalkylméthyle, un groupe phényle éventuellement substitué par un atome d'halogène, ou un groupe phénylméthyle éventuellement substitué par un atome d'halogène.

Dans la structure des composés de l'invention, l'un des atomes de carbone est asymétrique ; ils peuvent donc exister sous forme d'énantiomères optiquement purs ou de mélanges d'énantiomères. Par ailleurs ils peuvent se présenter à l'état de bases libres ou de sels d'addition.

Conformément à l'invention, les composés de formule générale (I) peuvent être préparés par réaction du α-(4-chlorophényl)-4-[(4-fluorophényl)méthyl]pipéridine-1-éthanol, de formule (II)

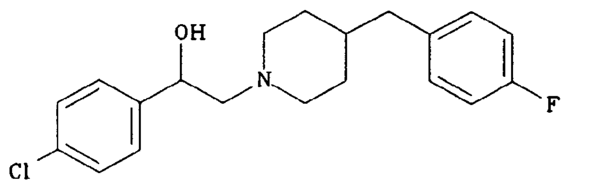

(II)

avec des anhydrides de formule générale $(RCO)_2O$ ou des chlorures d'acides de formule générale RCOCl, dans lesquelles R est tel que défini ci-dessus, dans les conditions de réaction bien connues pour des réactions d'estérification.

Le composé de formule (II) et ses énantiomères sont décrits dans les brevets EP-0109317 et FR-2628740.
Les chlorures d'acides de formule générale RCOCl sont disponibles dans le commerce ou bien peuvent être préparés à partir des acides correspondants selon toutes méthodes connues, par exemple par action du chlorure de thionyle.

Les exemples suivants illustrent en détail la préparation de quelques composés selon l'invention. Les microanalyses élémentaires et les spectres IR et RMN confirment les structures des composés obtenus.
Les numéros des composés indiqués entre parenthèses dans les titres correspondent à ceux du tableau donné plus loin.

<u>Exemple 1</u> (Composé N°1)

(*E*)-but-2-ènedioate d'acétate de (±)-α-(4-chlorophényl)-4-[(4-fluorophényl)méthyl]pipéridine-1-éthyle (1:1).

Dans un ballon de 250 ml on place 3,47 g (0,01 mole) de (±)-α-(4-chlorophényl)-4-[(4-fluorophényl)méthyl]pipéridine-1-éthanol et 20 ml d'anhydride acétique et on agite le mélange à température ambiante pendant une nuit.
On évapore l'excès d'anhydride, on reprend le résidu avec 100 ml d'acétate d'éthyle, on ajoute un excès d'ammoniaque 3N, on sépare la phase organique, on extrait la phase aqueuse avec de l'acétate d'éthyle, on réunit les phases organiques, on les lave à l'eau, on les sèche sur sulfate de sodium, on évapore le solvant sous pression réduite et on obtient 4,5 g de produit huileux qu'on purifie par chromatographie sur colonne de gel de silice en éluant avec un mélange 98/2 de dichlorométhane/méthanol.
On dissout la fraction purifiée dans l'éthanol et on prépare le fumarate avec un équivalent d'acide fumarique.
Après deux recristallisations dans l'éthanol et une recristallisation dans le propan-2-ol, puis séchage, on obtient finalement 0,89 g de composé.
Point de fusion : 164-165°C.

Exemple 2 (Composé N°2)

Acétate de *R*-(-)-α-(4-chlorophényl)-4-[(4-fluorophényl)méthyl]pipéridine-1-éthyle.

Dans un ballon de 50 ml on introduit 2,62 g (0,00753 mole) de *R*-(-)-α-(4-chlorophényl)-4-[(4-fluorophényl)méthyl] pipéridine-1-éthanol et 15 ml d'anhydride acétique, et on agite le mélange à température ambiante pendant une nuit. On verse le mélange sur de l'eau glacée, on agite pendant 1h, on ajoute de l'ammoniaque. Il se forme un produit huileux blanchâtre qui prend en masse. On le sépare et on le sèche en présence de pentoxyde de phosphore, ce qui donne 2,77 g de solide.
Après recristallisation dans le propan-2-ol et séchage on obtient 1,87 g de composé lévogyre.
Point de fusion : 73-74°C.

$[\alpha]_D^{25}$ = -43,8° (c=1 ; $CHCl_3$).

Exemple 3 (Composé N°3)

Acétate de *S*-(+)-α-(4-chlorophényl)-4-[(4-fluorophényl)méthyl]pipéridine-1-éthyle.

En utilisant la méthode décrite dans l'exemple précédent on obtient, à partir de 3,16 g (0,0098 mole) de *S*-(+)-α-(4-chlorophényl)-4-[(4-fluorophényl)méthyl]pipéridine-1-éthanol, 2,35 g de composé dextrogyre.
Point de fusion : 73-74°C.

$[\alpha]_D^{25}$ = +42,0° (c=1 ; $CHCl_3$).

Exemple 4 (Composé N°5)

(*E*)-but-2-ènedioate d'undécanoate de (±)-α-(4-chlorophényl)-4-[(4-fluorophényl)méthyl]pipéridine-1-éthyle (1:1).

On introduit dans un ballon 4,65 g (0,025 mole) d'acide undécanoïque, on ajoute 3,5 g (0,03 mole) de chlorure de thionyle, et on chauffe le mélange au bain d'huile à 60°C pendant 2h.
On évapore l'excès de chlorure de thionyle sous pression réduite, on en élimine les traces par entraînement au toluène, on refroidit le résidu par un bain de glace, on ajoute, goutte à goutte, une solution de 7 g (0,02 mole) de (±)-α-(4-chlorophényl)-4-[(4-fluorophényl)méthyl]pipéridine-1-éthanol dans 50 ml de pyridine, on agite le mélange jaune vif pendant 30 min, et on l'abandonne à température ambiante pendant une nuit.
On évapore le solvant sous pression réduite et on purifie le résidu par chromatographie sur colonne de gel de silice en éluant avec de l'acétate d'éthyle.
On obtient 4 g d'huile épaisse qu'on dissout dans 25 ml d'éthanol, on ajoute 1 g d'acide fumarique, et on place la solution au froid pendant 2j.
On ajoute 50 ml d'éther de pétrole à la masse cristallisée, on l'agite pendant 15 min et on sépare le précipité blanc par filtration. Après recristallisation dans le propan-2-ol on obtient 0,86 g de composé.
Point de fusion : 144-145°C.

Exemple 5 (Composé N°9)

(*E*)-but-2-ènedioate de cyclopentaneacétate de (±)-α-(4-chlorophényl)-4-[(4-fluorophényl)méthyl]pipéridine-1-éthyle (1:1).

Dans un ballon de 250 ml muni d'une garde à chlorure de calcium on place 3,8 g (0,03 mole) d'acide cyclopentaneacétique et 12 g (0,1 mole) de chlorure de thionyle, on ajoute une goutte de *N,N*-diméthylformamide, et on chauffe le mélange au bain d'huile à 60°C pendant 3h.
On agite le mélange à température ambiante pendant une nuit et on élimine l'excès de chlorure de thionyle par évaporation puis par entraînement au toluène.
On obtient une huile claire qu'on dilue avec 5 ml de toluène, on verse cette dernière, goutte à goutte, dans un ballon contenant 3,47 g (0,01 mole) de (±)-α-(4-chlorophényl)-4-[(4-fluorophényl)méthyl]pipéridine-1-éthanol en solution dans 15 ml de pyridine, et on agite le mélange à température ambiante pendant une nuit.
On évapore les solvants sous pression réduite, on élimine les traces de pyridine par entraînement à l'eau, on reprend le résidu avec de l'eau et de l'acétate d'éthyle, on sépare la phase organique, on extrait la phase aqueuse deux fois à

l'acétate d'éthyle, on réunit les phases organiques, on les sèche sur sulfate de sodium et on évapore le solvant sous pression réduite.

On obtient 7,9 g de produit huileux qu'on épuise avec de l'éther isopropylique, d'abord froid puis bouillant, on réunit les phases organiques, on élimine un louche par filtration sur papier, on évapore l'éther isopropylique sous pression réduite et on purifie le résidu par chromatographie sur colonne de gel de silice en éluant avec de l'éther isopropylique.

On obtient 3,14 g de produit huileux dont on prépare le fumarate dans l'éthanol avec un équivalent d'acide fumarique. Après recristallisation dans 25 ml de propan-2-ol et séchage on obtient finalement 1,94 g de fumarate.

Point de fusion : 146-147°C.

Le tableau qui suit illustre les structures chimiques et les propriétés physiques de quelques composés selon l'invention. Dans la colonne "R", "$cC_5H_9$-" désigne un groupe cyclopentyle et "4-F-$C_6H_4$-" désigne un groupe 4-fluorophényle.

Dans la colonne "Sel", "-" désigne un composé à l'état de base, "fum." désigne un (*E*)-but-2-ènedioate (1:1) (fumarate), "HCl" désigne un chlorhydrate (1:1) et "ox." désigne un éthanedioate (1:1) (oxalate).

Tableau

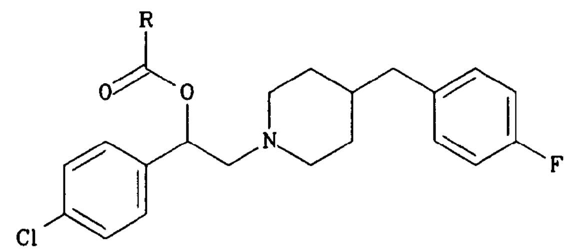

(I)

| N° | R | Isomère | Sel | F (°C) | $[\alpha]_D^{20}$ (c = 1) |
|---|---|---|---|---|---|
| 1 | $CH_3$- | (±) | fum. | 164-165 | |
| 2 | $CH_3$- | *R* (-) | - | 73-74 | -43,8° ($CHCl_3$) |
| 3 | $CH_3$- | *S* (+) | - | 73-74 | +42,0° ($CHCl_3$) |
| 4 | $CH_3-(CH_2)_5$- | (±) | fum. | 159-160 | - |
| 5 | $CH_3-(CH_2)_9$- | (±) | fum. | 144-145 | - |
| 6 | $(CH_3)_2CH$- | (±) | fum. | 173-174 | - |
| 7 | $(CH_3)_3C$- | (±) | fum. | 181-182 | - |
| 8 | $cC_5H_9$- | (±) | fum. | 169-170 | - |

| N° | R | Isomère | Sel | F (°C) | $[\alpha]_D^{20}$ (c = 1) |
|---|---|---|---|---|---|
| 9 | $cC_5H_9-CH_2-$ | (±) | fum. | 146-147 | - |
| 10 | $4-F-C_6H_4-$ | (±) | fum. | 197-198 | - |
| 11 | $4-F-C_6H_4-CH_2-$ | (±) | HCl | 195-196 | - |
| 12 | $CH_3(CH_2)_{16}-$ | (±) | fum. | 115-116 | - |
| 13 | $CH_3(CH_2)_{16}-$ | *R* (-) | fum. | 116-118 | -28,9° ($CH_3OH$) |
| 14 | $CH_3(CH_2)_{16}-$ | *S* (+) | fum. | 116-118 | +26,7° ($CH_3OH$) |
| 15 | *cis*-$CH_3(CH_2)_7CH=CH(CH_2)_7-$ | (±) | ox. | 119-120 | - |
| 16 | *cis*-$CH_3(CH_2)_7CH=CH(CH_2)_7-$ | *R* (-) | ox. | 99-100 | -27,5° ($CH_3OH$) |
| 17 | *cis*-$CH_3(CH_2)_7CH=CH(CH_2)_7-$ | *S* (+) | ox. | 99-100 | +26,5° ($CH_3OH$) |

Les composés de l'invention ont été soumis à des essais qui ont mis en évidence leur activité neuroprotectrice et neurotrophe.

Ainsi ils ont fait l'objet d'un essai d'inhibition de la liaison du [$^3$H]ifenprodil aux sites modulateurs sensibles aux polyamines du complexe récepteur NMDA dans les membranes de cortex cérébral de rat, d'après le protocole décrit par Schoemaker et col., *Eur. J. Pharmacol.* (1990) **176** 249-250. Les rats mâles Sprague-Dawley de 150 à 230 g sont sacrifiés et le cortex cérébral est homogénéisé dans 20 volumes de tampon Tris-HCl à 50mM (pH=7,4 à 0°C) glacé, au moyen d'un appareil Ultra-Turax™ (Ikawerk) ou Polytron™ (Kinematica). L'homogénat est lavé deux fois par centrifugation pendant 10 min à 45000xg, le culot étant remis en suspension dans du tampon frais. Le culot final est repris dans 20 volumes du même tampon.

Une aliquote de 100 µl de cette suspension est incubée dans un volume final de 1000 µl avec 1 nM de [$^3$H]ifenprodil

(activité spécifique : 30 à 35 Ci/mmole) pendant 120 min à 0°C, en présence de 3 µM de GBR 12909 (Research Biochemicals Inc., Natick, MA, USA), en l'absence ou en présence de substance compétitrice.
Après incubation, le mélange est dilué avec 5 ml de tampon Tris-HCl à 50mM (pH=7,4 à 0°C) glacé et les membranes sont récupérées par filtration sur des filtres Whatman GF/B™ prétraités avec de la polyéthylèneimine à 0,05%, puis lavées avec deux fois 5 ml de tampon glacé.
On détermine la liaison non spécifique avec l'ifenprodil 10µM, on analyse les données selon les méthodes usuelles, et on calcule la concentration $CI_{50}$, concentration qui inhibe de 50% la liaison du [$^3$H]ifenprodil.
Les $CI_{50}$ des composés les plus actifs dans cet essai sont de l'ordre de 0,4 µM.

L'affinité des composés de l'invention pour un site de liaison du [$^3$H]ifenprodil, non associé aux récepteurs NMDA et présentant des analogies avec les sites $\sigma_2$, a également été évaluée.

Les rats mâles Sprague-Dawley, d'un poids de 150 à 230 g, sont sacrifiés et le cortex cérébral est homogénéisé à 4°C dans 20 volumes de tampon Tris-HCl 50 mM, pH=7,4, glacé,au moyen d'un appareil Ultra-Turax™ (Ikawerk) ou Polytron™ (Kinematica). Après centrifugation pendant 10 min à 45000xg, le surnageant est éliminé et le culot lavé une nouvelle fois dans les mêmes conditions, puis remis en suspension dans le volume de tampon de départ.
Des aliquotes de 100 µl de suspension de membranes sont incubées pendant 30 min à 37°C dans 1 ml de tampon contenant les composés à tester et 0,5 nM de [$^3$H]ifenprodil. La liaison non-spécifique est déterminée en présence de 10 µM d'ifenprodil. La radioactivité liée est séparée par filtration sur filtres Whatman GF/B™ préalablement traités par de la polyéthylèneimine à 0,05%, puis lavés deux fois avec 5 ml de tampon glacé. Les résultats sont analysés selon les méthodes usuelles, et on calcule la concentration qui inhibe de 50% la liaison du [$^3$H]ifenprodil.
Les $CI_{50}$ des composés les plus actifs dans ce test vont de 0,04 à 0,4 µM.

Enfin les composés de l'invention ont été soumis au test de l'ischémie cérébrale globale chez la souris. L'ischémie est provoquée par un arrêt cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.
L'arrêt respiratoire apparaît approximativement 19 secondes après l'injection de chlorure de magnésium chez les souris témoins.
Des souris mâles (Charles River CD1) sont étudiées par groupes de 10. Elles sont nourries et abreuvées ad libitum avant les essais. Le temps de survie est mesuré 10 min après l'administration intrapéritonéale des composés de l'invention. On calcule la différence entre le temps de survie mesuré dans un groupe de 10 souris ayant reçu le composé à étudier et le temps de survie mesuré dans un groupe de 10 souris ayant reçu le liquide véhicule.
Les allongements du temps de survie en fonction de la dose du composé sont exprimés graphiquement selon une courbe semilogarithmique.
Cette courbe permet le calcul de la "dose efficace 3 secondes" ($DE_{3''}$), c'est-à-dire la dose (en mg/kg) qui produit une augmentation de 3 secondes du temps de survie par rapport au groupe témoin de souris non traitées.
Une augmentation de 3 secondes du temps de survie est à la fois significative statistiquement et reproductible.
Les $DE_{3''}$ des composés les plus actifs dans cet essai sont de l'ordre de 3 mg/kg par voie intrapéritonéale.

Les résultats des essais effectués suggèrent que les composés de l'invention possèdent des activités neuroprotectrices et neurotrophes.
Ces activités peuvent trouver une application dans le traitement et la prévention des désordres neurologiques tels que ceux qui sont consécutifs, par exemple, à une attaque ischémique, un arrêt cardiaque ou respiratoire, une thrombose ou une embolie cérébrale, un traumatisme crânien ou médullaire. Les composés de l'invention peuvent aussi être utilisés pour le traitement de la sénilité cérébrale, de la démence consécutive aux infarctus multiples, de la démence vasculaire, de la sclérose en plaques, pour le traitement de l'atrophie olivo-ponto-cérébelleuse et d'autres maladies neurodégénératives, par exemple la maladie d'Alzheimer, la maladie de Pick, la chorée de Huntington.
Ils sont utilisables également pour le traitement des neuropathies périphériques de type traumatique, ischémique, métabolique, infectieux, alcoolique, iatrogénique ou génétique, pour le traitement des maladies affectant les motoneurones, comme la sclérose latérale amyotrophique et les amyotrophies.
Chez les malades atteints de glaucome ils peuvent servir à la prévention de la dégénérescence du nerf optique ou de la rétine.
Enfin, on peut envisager leur utilisation dans le traitement des états convulsifs, le traitement de la migraine, le traitement des états d'accoutumance et/ou d'addiction aux analgésiques narcotiques, et comme antiémétiques.

Les composés de l'invention peuvent être utilisés seuls ou en association avec d'autres substances thérapeutiques, par exemple avec un agent thrombolytique, tel qu'un activateur tissulaire du plasminogène recombiné, pour le traitement des infarctus cérébraux de type thrombo-embolique, ou avec un composé diminuant la pression intraoculaire, pour le traitement du glaucome, ou encore avec un agent anticancéreux, en vue de réduire les effets secondaires (neuropathies et autres) de ce dernier.

A cet effet ils peuvent être présentés sous toutes formes pharmaceutiques adaptées à l'administration entérale ou

parentérale, en association avec des excipients appropriés, par exemple sous forme de comprimés, dragées, gélules, capsules, suppositoires, patches, solutions ou suspensions buvables ou injectables, dosés pour permettre une administration journalière de 1 à 1000 mg de substance active.

## Revendications

1. Composé, sous forme d'énantiomère optiquement pur ou de mélange d'énantiomères, répondant à la formule générale (I)

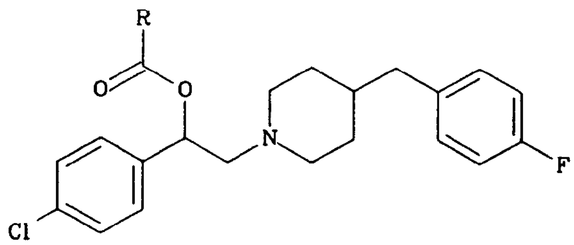

(I)

dans laquelle R représente un groupe ($C_1$-$C_{19}$)alkyle linéaire ou éventuellement ramifié et/ou contenant éventuellement une liaison éthylénique, un groupe ($C_3$-$C_6$)cycloalkyle, un groupe ($C_3$-$C_6$)cycloalkylméthyle, un groupe phényle éventuellement substitué par un atome d'halogène, ou un groupe phénylméthyle éventuellement substitué par un atome d'halogène,
à l'état de base libre ou de sel d'addition.

2. Procédé de préparation d'un composé selon la revendication 1, caractérisé en ce qu'on fait réagir le α-(4-chlorophényl)-4-[(4-fluorophényl)méthyl]pipéridine-1-éthanol avec un anhydride de formule générale $(RCO)_2O$ ou un chlorure d'acide de formule générale RCOCl, dans lesquelles R est tel que défini dans la revendication 1.

3. Médicament caractérisé en ce qu'il consiste en un composé selon la revendication 1.

4. Composition pharmaceutique caractérisée en ce qu'elle contient un composé selon la revendication 1, associé à un excipient.

Office européen des brevets

RAPPORT DE RECHERCHE EUROPEENNE

Numero de la demande
EP 95 40 1658

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | EP-A-0 109 317 (SYNTHELABO) 23 Mai 1984<br>* le document en entier *<br>--- | 1-4 | C07D211/18<br>A61K31/445 |
| D,A | FR-A-2 628 740 (SYNTHELABO) 22 Septembre 1989<br>* page 2, ligne 10 - ligne 27 *<br>--- | 1-4 | |
| A | FR-A-2 694 555 (SYNTHELABO) 11 Février 1994<br>* le document en entier *<br>----- | 1-4 | |
| | | | DOMAINES TECHNIQUES RECHERCHES (Int.Cl.6) |
| | | | C07D |

Le présent rapport a été établi pour toutes les revendications

| Lieu de la recherche | Date d'achèvement de la recherche | Examinateur |
|---|---|---|
| LA HAYE | 10 Octobre 1995 | Kissler, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)